(19) Europäisches Patentamt
European Patent Office
Office européen des brevets

(11) Veröffentlichungsnummer: **0 356 944 A1**

# (12) EUROPÄISCHE PATENTANMELDUNG

(21) Anmeldenummer: **89115760.4**

(22) Anmeldetag: **26.08.89**

(51) Int. Cl.5: **C07D 493/10 , A61K 31/35**

(30) Priorität: **31.08.88 DE 3829450**

(43) Veröffentlichungstag der Anmeldung:
**07.03.90 Patentblatt 90/10**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim (Taunus)(DE)**
Erfinder: **Seibert, Gerhard, Prof. Dr.**
**Gläserweg 21**
**D-6100 Darmstadt(DE)**
Erfinder: **Winkler, Irvin, Dr.**
**In den Eichen 40**
**D-6237 Liederbach(DE)**
Erfinder: **Grabley, Susanne, Dr.**
**Hölderlinstrasse 7**
**D-6240 Königstein/Taunus(DE)**

## (54) Nigericinderivate, Verfahren zu deren Herstellung, diese enthaltende Mittel und Verwendung derselben.

(57) Die Erfindung betrifft Nigericinderivate der Formel I

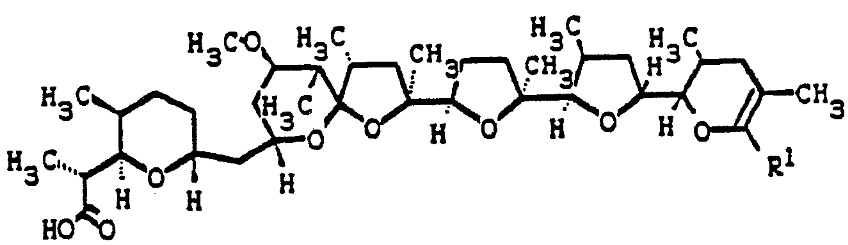

(I)

in der $R^1$ die angegebenen Bedeutungen hat. Die Verbindungen zeigen antivirale und antibakterielle Wirksamkeit.

EP 0 356 944 A1

# Nigericinderivate, Verfahren zu deren Herstellung, diese enthaltende Mittel und Verwendung derselben

Der Polyether Nigericin, der durch Kultivierung von Streptomyces hygroscopicus erhalten werden kann, ist u. a. beschrieben in J. Berger et al., Am. Chem. Soc. 73, 5295 (1951)

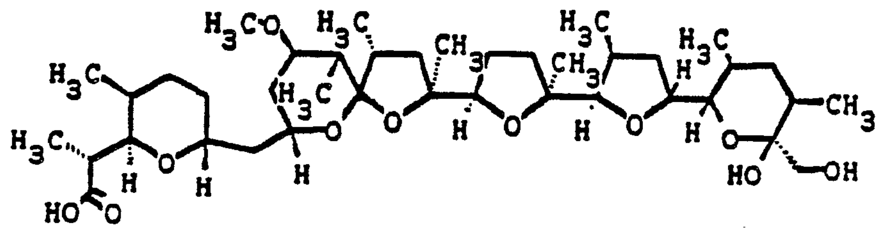

und wurde bislang als Antibiotikum eingesetzt.

Einige Derivate des Nigericins sind ebenfalls bekannt (JP 72 01,288 und T. Kubota, J. Chem. Soc. (C) 1970, 695, US 3,995,027), auch sie wurden bislang als Antibiotika oder gegen Tierviren eingesetzt. Ebenfalls bekannt ist ein Nigericin-Ester der Formel.

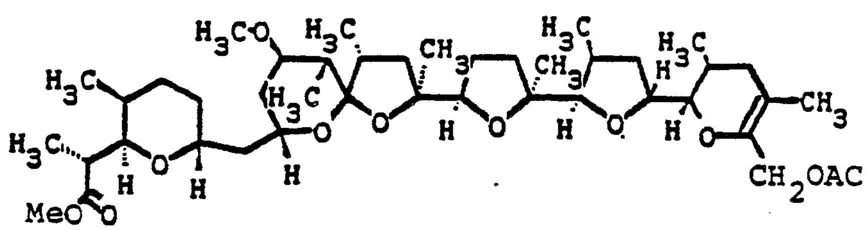

Eine antivirale Wirksamkeit von Nigericin oder Nigericinderivaten, insbesondere gegen Viren, die bei Menschen Erkrankungen hervorrufen können, ist bisher noch nicht bekannt geworden. Lediglich in den älteren deutschen Anmeldungen P 38 00 598, P 38 11 016 und P 38 20 179.8 ist eine solche antivirale Wirksamkeit vorgeschlagen worden; diese ist jedoch in vielerlei Hinsicht noch nicht befriedigend.

Es wurde nun gefunden, daß bestimmte Nigericinderivate hochwirksame antiviral und antibakteriell wirksame Substanzen sind.

Die Erfindung betrifft nun

1. Nigericinderivate der Formel I

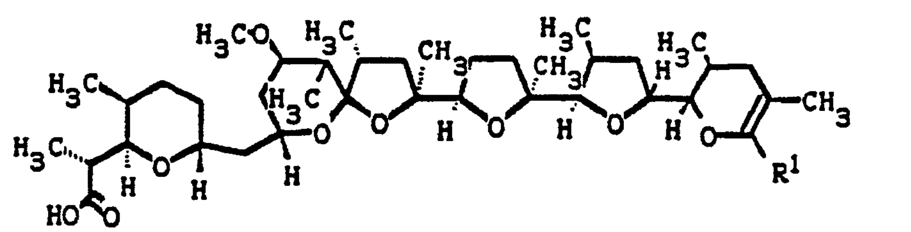

(I)

in der

$R^1$ CHO oder $CH_2$-O-$R^2$ bedeutet, wobei

$R^2$ Wasserstoff bedeutet oder einen Rest der Formel

$-\overset{O}{\overset{\|}{C}}-R^3$, $-\overset{O}{\overset{\|}{C}}-NH-R^3$ oder $-\overset{O}{\overset{\|}{C}}-O-R^3$ darstellt, in der

$R^3$ $C_1$-$C_{15}$-Alkyl bedeutet, welches gegebenenfalls halogen-, nitro, cyano-, carboxyl-, $C_1$-$C_4$-alkoxy-, phenyloxy- oder phenylsubstituiert ist, wobei die genannten Phenylreste ihrerseits mit Halogen, Nitro, Cyano, $C_1$-$C_4$-Alkyl oder $C_1$-$C_4$-Alkoxy substituiert sein können, oder in der

$R^3$ Phenyl, Furyl oder Thienyl bedeutet, wobei diese Phenyl-, Furyl- oder Thienyl-Reste mit Halogen, Nitro, Cyano, $C_1$-$C_4$-Alkyl oder $C_1$-$C_4$-Alkoxy substituiert sein können,

sowie die physiologisch verträglichen Salze dieser Verbindungen.

2. Die vorgenannten Verbindungen zur Anwendung als Arzneimittel.
3. Die vorgenannten Verbindungen zur Anwendung als antibakteriell wirksame Arzneimittel.
4 Die vorgenannten Verbindungen, zur Anwendung als antiviral wirksame Arzneimittel.
5. Ein Verfahren zur Herstellung der Verbindungen der Formel I, wobei man

a) Nigericin einer Eliminierungsreaktion unterwirft, wobei eine Verbindung der Formel II entsteht

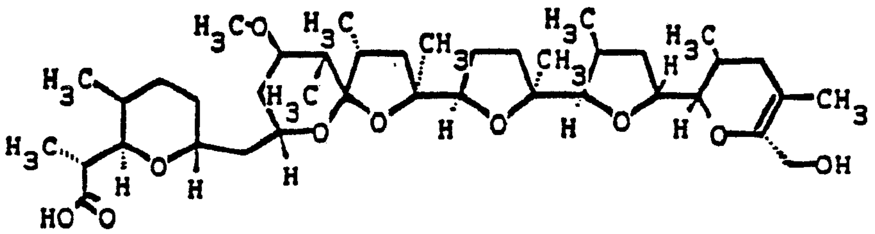

(II)

und daß man dann gegebenenfalls

b) das Nigericinderivat der Formel II umsetzt mit einer Verbindung der Formel III oder IV

$X-\overset{O}{\overset{\|}{C}}-R^3$ (III) $X-\overset{O}{\overset{\|}{C}}-O-R^3$ (IV)

worin
X Chlor, Brom oder ein Anhydrid bedeutet und
$R^3$ die oben zu Formel I angegebenen Bedeutungen hat, oder daß man gegebenenfalls

c) das Nigericinderivat der Formel II oxidiert zu der Verbindung der Formel V

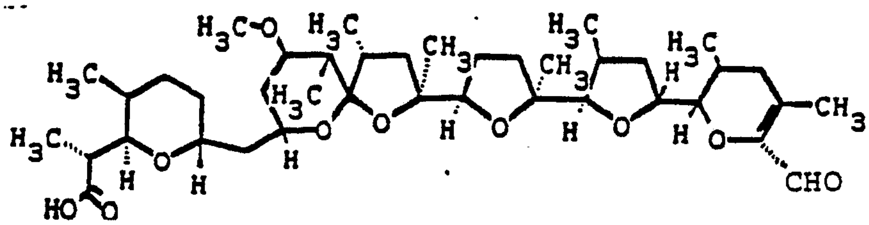

(V)

und daß man gegebenenfalls anschließend die Verbindungen der Formel I in ihre physiologisch verträglichen Salze überführt.

Unter Halogenen werden Fluor, Chlor, Brom und Jod verstanden, insbesondere Chlor und Brom.

Alkylgruppen mit mehr als 2 Kohlenstoffatomen können sowohl geradkettig als auch verzweigt sein.

Im folgenden werden die Verfahren a) bis c), die es ermöglichen, die unterschiedlich substituierten Nigericinderivate herzustellen, näher beschrieben.

Bei der Verfahrensvariante a) geht man am besten so vor, daß man Nigericin einer Eliminierungsreaktion unterwirft, wobei eine Verbindung der Formel II entsteht. Dies kann z.B. durch eine Dehydrohalogenierung bzw. eine Detosylierung der p-Toluolsulfonsäureester geschehen. Solche Verfahren werden beispielsweise beschrieben im ORGANIKUM, organisch-chemisches Grundpraktikum, 15. Auflage, Seiten 275-307, VEB Deutscher Verlag der Wissenschaften, Berlin 1976.

Bevorzugt ist die Detosylierung. Hierzu wird Nigericin mit 0,1-1 bevorzugt 0,3-0,6 Moläquivalenten (bezogen auf die eingesetzt Menge an Nigericin) p-Toluolsulfonsäure in einem inerten, aprotischen Lösungsmittel wie Chloroform, Methylenchlorid, Tetrahydrofuran (THF), Essigester oder Dioxan 12 bis 72, bevorzugt 12-48 Stunden erhitzt. Die Reaktionstemperaturen liegen dabei zwischen 40 und 120 °C, bevorzugt bei der Siedetemperatur des verwendeten Lösungsmittels. Die Beendigung der Reaktion kann beispielsweise mittels Dünnschichtchromatographie bestimmt werden.

Das als Ausgangssubstanz benötigte Nigericin kann beispielsweise nach dem von J. Berger (loc. cit. Am. Chem. Soc.) beschriebenen Verfahren hergestellt werden. Es kann aber auch gemäß dem in der deutschen Patentanmeldung P 3700325.9 vorgeschlagenen Verfahren hergestellt werden, wobei Nigericin neben Amycin bei der Kultivierung von Streptomyces parvulus DSM 3816 entsteht. Das Nigericin kann aus dem Mycel mittels Hexan extrahiert und nach Aufkonzentrierung auskristallisiert werden.

Bei der Verfahrensvarianten b) geht man am besten so vor, daß man das Nigericinderivat der Formel II in äquimolaren Mengen oder in bis zu einem 50-fachen Überschuß, gegenbenenfalls in einem inerten, aprotischen Lösungsmittel wie Chloroform, Methylenchlorid, Tetrahydrofuran (THF), Essigester oder Dioxan, mit einer Verbindung der Formel III oder IV insbesondere den Anhydriden - bis zur Beendigung der

Reaktion umsetzt, gegebenenfalls in Gegenwart einer Base, vorzugsweise Pyridin oder DMAP (Dimethylaminopyridin).

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C.

Die Reaktionszeiten betragen 1 bis 180 Stunden, bevorzugt 1 bis 48 Stunden. Die Beendigung der Reaktion kann beispielsweise mittels Dünnschichtchromatographie bestimmt werden (DE-Kontrolle).

Die Ausgangsverbindungen für die Verfahrensvariante b), die Verbindungen der Formel III oder IV sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter de Gruyter, Berlin, New York 1982, S. 303 ff. (Zur Herstellung der Anhydride siehe beispielsweise: ORGANIKUM, Organisch-Chemisches Grundpraktikum, 15. Auflage (1976), VEB Deutscher Verlag der Wissenschaften, Berlin, Methodenregister S. 824: Carbonsäureanhydride).

Bei der Verfahrensvarianten c) geht man am besten so vor, daß man den primären Alkohol der Formel II mit einem Oxidationsmittel wie Chromsäure, Bichromat/Schwefelsäure, Salpetersäure, Braunstein ($MnO_2$) oder Selendioxid zu der Verbindung der Formel V umsetzt. Solche Verfahren sind beispielsweise beschrieben im ORGANIKUM, organisch-chemisches Grundpraktikum, 15. Auflage, S. 443-447, VEB Deutscher Verlag der Wissenschaften, Berlin 1976. Bevorzugt ist die Oxidation mit Braunstein ($MnO_2$). Hierzu wird das Nigericinderivat der Formel II mit 5-20, bevorzugt 8-12 Moläquivalenten (bezogen auf die eingesetzte Menge an Nigericinderivat der Formel II) Mangandioxid in einem inerte Lösungmittel wie Chloroform, Toluol, THF oder Dioxan versetzt. Die Reaktionstemperatur liegt zwischen 20 °C und dem Siedepunkt, bevorzugt bei Raumtemperatur. Die Reaktionszeiten liegen bei 10-100, bevorzugt bei 10-30 Stunden. Die Beendigung der Reaktion kann beispielsweise mittels Dünnschichtchromatographie bestimmt werden.

Die Herstellung der physiologisch verträglichen Salze erfolgt nach an sich bekannten Methoden durch Umsetzung mit anorganischen oder organischen Basen. Zur Salzbildung sind insbesondere geeignet Alkali- und Erdalkalihydroxyde, -carbonate oder -bicarbonate sowie physiologisch verträgliche organische Verbindungen, die eine primäre, sekundäre oder tertiäre Aminogruppe tragen.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform oder Essigester oder Methanol/Chloroform-Mischungen, aber auch extraktive Methoden wie flüssig/flüssig-Extraktion oder fest/flüssig-Extraktion oder durch Kristallisation gereinigt werden.

Die erfindungsgemäßen Nigericinderivate zeigen ausgezeichnete antivirale Wirkung. Diese antivirale Aktivität wurde in Zellkulturen, die mit Testviren infiziert wurden, getestet. Ebenso zeigen die Derivate eine antibakterielle Wirkung.

Die erfindungsgemäßen Verbindungen sind aufgrund ihrer pharmakologischen Eigenschaften für die Behandlung von bakteriellen Erkrankungen und Viruserkrankungen, die hervorgerufen werden beispielsweise durch HSV I, II (Herpes simplex I- oder II-Virus) oder auch Picorna- und Retroviren, wie HIV (Human Immunodeficiency Virus) geeignet.

Die Erfindung betrifft daher weiter die Anwendung der erfindungsgemäßen Verbindungen der Formel I sowie deren physiologisch verträgliche Salze bei der Behandlung und Prophylaxe von bakteriellen Erkrankungen oder von Herpes-Virus-, Picorna- und Retroviren-Erkrankungen.

Die neuen Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tab, vorzugsweise 0,001 -1,0 mg/kg/Tag, inbesondere 0,005 - 0,2 mg/kg/Tag, appliziert werden. Besonders bevorzugt ist die topische Anwendung, wobei die Wirkstoffkonzentration in den Salben 0,001 - 1 %, bevorzugt 0,01 - 0,1 %, beträgt. Die Dosierung kann in schweren Fällen auch erhöht werden. In vielen Fällen genügen jedoch auch geringere Dosen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I enthalten.

Die Arzneimittel werden nach an sich bekannten, dem Fachmann geläufigen Verfahren hergestellt. Als Arzneimittel werden die erfindungsgemäßen pharmakoligisch wirksamen Verbindungen (-Wirkstoff) entweder als solche oder vorzugsweise in Kombination mit geeigneten pharmazeutischen Hilfs- oder Trägerstoffen in

Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Bei der topischen Anwendung reichen schon Wirkstoffkonzentrationen von 0,001 - 1 %, bevorzugt 0,01 -0,1 %, aus.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral (subkutan), topisch oder rectal appliziert werden, wobei die topische Applikation bevorzugt ist.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen, Cremes oder Salben. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung anhand von Beispielen näher erläutert.

**Allgemeine Verfahrensvorschriften**

**Verfahren 1**

725 mg (1 mmol) Nigericin wird in 40 ml wasserfreiem Methylenchlorid mit 50 mg para-Toluolsulfonsäure für 28 Stunden unter Rückfluß gerührt. Nach der Extraktion mit 0,1 N $Na_2CO_3$ und 0,1 N HCl wird die organische Phase im Vakuum getrocknet. Nach dem Einrotieren wird der verbleibende Rückstand an Kieselgel chromatographiert ($CHCl_3$/MeOH 40 : 1).

**Verfahren 2**

725 mg (1 mmol) Nigericinderivat der Formel II, erhältlich wie unter Verfahren 1 beschrieben, werden in 10 ml Pyridin gelöst und mit 1,5 mmol Anhydrid in Gegenwart von 10 mg Dimethylaminopyridin für 4 - 24 h umgesetzt. Nach der Zugabe von 40 ml Wasser wird weitere 20 min bei Raumtemperatur gerührt. Die wäßrige Phase wird mit Ethylacetat (3 x 20 ml) extrahiert und die organische Phase wird mit 2 x 10 ml 0,1 N HCl und 2 x 10 ml Wasser gewaschen. Nach dem Trocknen über Natriumsulfat wird im Vakuum einrotiert. Chromatographie an 250 g Kieselgel mit Chloroform/Methanol von 30 : 1 auf 9 : 1 ergibt die Ester.

**Verfahren 3**

1 mmol der gemäß Verfahren 1 erhältlichen Substanz wird in 20 ml Toluol mit 800 mg $MnO_2$ für 20 Stunden bei Raumtemperatur gerührt. Nach dem Abfiltrieren wird der Rückstand an Kieselgel chromatographiert ($CHCl_3$/MeOH 30 : 1).

In den nachfolgenden Tabellen sind die gemäß den vorstehend beschriebenen Verfahren 1 bis 3 synthetisierten Verbindungen aufgeführt. In Tabelle 1 ist der jeweilige Substituent $R^1$ der Nigericinderivate zwecks Identifizierung der Verbindungen angegeben. Ebenfalls angegeben in Tabelle 1 ist das Molekulargewicht, die Reaktionszeit, die Reaktionstemperatur, das Herstellungsverfahren und die chemische Ausbeute. In Tabelle 2 sind ausgewählte, charakteristische analytische Daten der erhaltenen Verbindungen angegeben

(Molmassenpeak der Natriumverbindung im Massenspektrum ($MNa^+$) und $^{13}C$-NMR-Daten).

**Tabelle 1**

| Bsp. Nr. | $R^1$ | Molgewicht | Verfahren | Ausbeute | Zeit [Std.] | Temperatur [°C] |
|---|---|---|---|---|---|---|
| 1 | $CH_2OH$ | 707 | 1 | 72 % | | |
| 2 | $CH_2-O-\overset{O}{\overset{\|}{C}}-CH_3$ | 749 | 2 | 84 % | 24 | 25 |
| 3 | 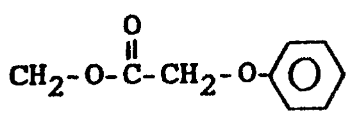 | 841,1 | 2 | 90 % | 24 | 25 |
| 4 | $CH_2-O-\overset{O}{\overset{\|}{C}}-CH_2-CH_2-COOH$ | 807,6 | 2 | 92 % | 24 | 25 |
| 5 | 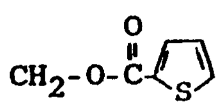 | 817 | 2 | 88 % | 24 | 25 |
| 6 | CHO | 705 | 3 | 62 % | 16 | 25 |

Tabelle 2

| Bsp. Nr. | MNa$^+$(FABMS) | $^{13}$C-NMR (ppm) | | | | |
|---|---|---|---|---|---|---|
| 1 | 729 | 181,7 | 146,3 | 107,7 | 100,6 | |
| 2 | 771 | 176,8 | 170,8 | 141,3 | 107,7 | 107,5 |
| 3 | 863 | 176,8 | 168,8 | 157,9 | 140,9 | 129,4 |
| | | 121,6 | 114,7 | 108,2 | 107,7 | |
| 4 | 829 | | | | | |
| 5 | 839 | | | | | |
| 6 | 727 | | | | | |

**Antivirale Wirksamkeit**

Antivirale Aktivität in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgte die Zugabe von 100 µl einer Suspension von HeLa- bzw. Vero-Zellen in Medium mit 5 % fötalem Kälberserum in einer Zelldichte von 2 x $10^5$ Zellen/ml. Die Ansätze wurden mit 50 µl einer Suspension des jeweiligen Test-Virus infiziert, die so eingestellt war, daß die Zellen innerhalb von 72 h einen cytopathogenen Effekt (CPE) zeigten. Die Auswertung erfolgte durch mikroskopische Begutachtung des Zellrasens und photometrische Messung der Neutralrotaufnahme (Farbtest nach Finter). Als MHK wurde die Konzentration des Präparats angenommen (µg/ml), bei der etwa 50 % der Zellen die Infektion überlebten.

In Tabelle 3 ist die Wirkung (Angabe des MHK in µg/ml) von verschiedenen erfindungsgemäßen Verbindungen gegen folgende Viren aufgeführt:
Adeno 5, Vaccina, Herpes I, Herpes II, Influenza A, Paramyxo. III, Rhinovirus II.

Tabelle 3

| MHK in µg/ml | | | | | | | |
|---|---|---|---|---|---|---|---|
| Verb. aus Bsp. | Adeno 5 | Vaccina | Herpes I | Herpes II | Influenza A | Paramyxo. III | Rhino. II |
| 1 | < 0,02 | < 0,02 | < 0,02 | 0,05 | 1,98 | -- | 1,48 |
| 2 | < 0,02 | < 0,02 | < 0,02 | 1,98 | 0,49 | -- | 1,48 |
| 3 | < 0,02 | < 0,02 | < 0,02 | 0,16 | 1,48 | -- | 4,44 |
| 4 | 0,06 | < 0,02 | < 0,02 | 4,44 | 0,49 | -- | 4,44 |

**Antibakterielle Wirksamkeit**

Die antibakterielle Wirksamkeit der erfindungsgemäßen Verbindungen wurde im Agar-Verdünnungstest nach Lorian (Antibiotics in Laboratory Medicine, Williams & Wilkins, Baltimore/London, 1980) bestimmt. Dabei wurden die Präparate in einer geometrischen Verdünnungsreihe mit dem Faktor 2 in Müller Hinton Agar verdünnt. Eine Petrischale enthielt nur Müller Hinton Agar und diente zur Kontrolle des Bakterienwachstums. Die Petrischalen wurden dann mit einem Denley-Multipoint-Inokulator, welcher 0,6 µl einer 1 : 100 verdünnten 18-Stunden-Kultur der Testbakterien übertrug, mit den entsprechenden Testbakterien beimpft. Nach 16 bis 18 Stunden Inkubation bei 37°C wurden die Petrischalen makroskopisch auf Bakterienwachstum untersucht.

Die niedrigste Konzentration der erfindungsgemäßen Verbindungen, welche das Bakterienwachstum noch vollständig verhinderte, wurde als MIC (Minimum Inhibitory Concentration) angenommen.

In Tabelle 4 ist die Wirkung (Angabe des MIC in µg/ml) von verschiedenen erfindungsgemäßen Verbindungen gegen folgende Bakterien aufgeführt:
Staph. aureus (SG 511), Staph. aureus (285),
Staph. aureus (503), Strept. pyogenes (308 A),
Strept. pyogenes (77 A), Strept. faecium (D).

Tabelle 4

| MIC in µg/ml | | | | | | |
|---|---|---|---|---|---|---|
| Verb. aus Bsp. | Staph. aureus (SG 511) | Staph. aureus (285) | Staph. aureus (503) | Strept. pyogenes (308 A) | Strept. pyogenes (77 A) | Strept. faecium (D) |
| 1 | 1,56 | 0,78 | 0,39 | 0,098 | 0,049 | 1,56 |
| 2 | 3,13 | 3,13 | 1,56 | 0,39 | 0,39 | 3,13 |
| 3 | 3,13 | 6,25 | 1,56 | 0,098 | 0,195 | 6,25 |
| 4 | 12,5 | 12,5 | 12,5 | 1,56 | 1,56 | 25,0 |

## Ansprüche

1. Nigericinderivate der Formel I

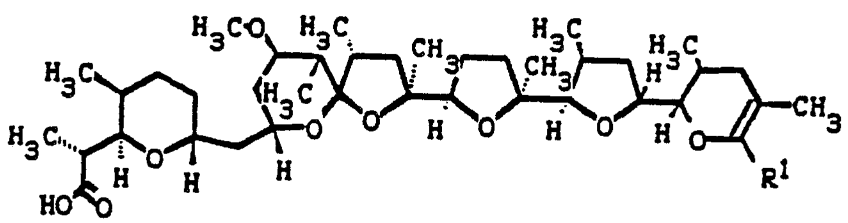

(I)

in der
$R^1$ CHO oder $CH_2$-O-$R^2$ bedeutet, wobei
$R^2$ Wasserstoff bedeutet oder einen Rest der Formel
$-\overset{O}{\overset{\|}{C}}-R^3$, $-\overset{O}{\overset{\|}{C}}-NH-R^3$ oder $-\overset{O}{\overset{\|}{C}}-O-R^3$ darstellt, in der
$R^3$ $C_1$-$C_{15}$-Alkyl bedeutet, welches gegebenenfalls halogen-, nitro-, cyano-, carboxyl-, $C_1$-$C_4$-alkoxy-, phenyloxy- oder phenylsubstituiert ist, wobei die genannten Phenylreste ihrerseits mit Halogen, Nitro, Cyano, $C_1$-$C_4$-Alkyl oder $C_1$-$C_4$-Alkoxy substituiert sein können, oder in der
$R^3$ Phenyl, Furyl oder Thienyl bedeutet, wobei diese Phenyl-, Furyl- oder Thienyl-Reste mit Halogen, Nitro, Cyano, $C_1$-$C_4$-Alkyl oder $C_1$-$C_4$-Alkoxy substituiert sein können,
sowie deren physiologisch verträgliche Salze.

2. Nigericinderivate nach Anspruch 1, dadurch gekennzeichnet, daß mindestens eine der nachfolgenden Bedingungen erfüllt ist:
$R^2$ bedeutet Wasserstoff oder einen Rest der Formel
$-\overset{O}{\overset{\|}{C}}-R^3$
$R^3$ bedeutet $C_1$-$C_8$-Alkyl, welches gegebenenfalls carboxyl- oder phenyloxysubstituiert ist, oder
$R^3$ bedeutet Furyl oder Thienyl.

3. Nigericinderivate nach Anspruch 1 und/oder 2, dadurch gekennzeichnet, daß mindestens eine der nachfolgenden Bedingungen erfüllt ist:
$R^2$ bedeutet Wasserstoff oder einen Rest der Formel
$-\overset{O}{\overset{\|}{C}}-R^3$
$R^3$ bedeutet $C_1$-$C_4$-Alkyl, welches gegebenenfalls carboxyl- oder phenyloxysubstituiert ist, oder
$R^3$ bedeutet Thienyl.

4. Verbindungen nach einem oder mehreren der Ansprüche 1 bis 3 zur Anwendung als Arzneimittel.

5. Verbindungen nach einem oder mehreren der Ansprüche 1 bis 3 zur Anwendung als antibakteriell wirksame Arzneimittel.

6. Verbindungen nach einem oder mehreren der Ansprüche 1 bis 3 zur Anwendung als antiviral wirksame Arzneimittel.

7. Verfahren zur Herstellung von Verbindungen der Formel I, dadurch gekennzeichnet, daß man

a) Nigericin einer Eliminierungsreaktion unterwirft, wobei eine Verbindung der Formel II entsteht

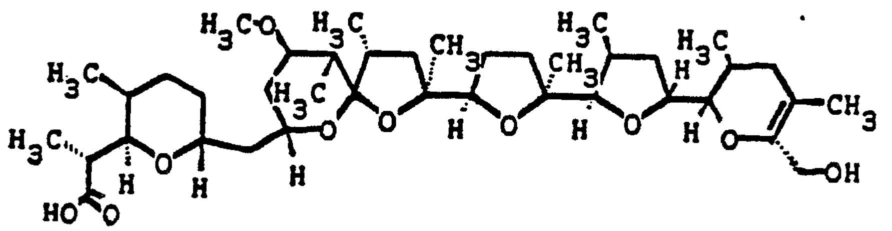

(II)

und daß man dann gegebenenfalls

b) das Nigericinderivat der Formel II umsetzt mit einer Verbindung der Formel III oder IV

$X-\overset{O}{\overset{\|}{C}}-R^3$ (III) $X-\overset{O}{\overset{\|}{C}}-O-R^3$ (IV)

worin

X Chlor, Brom oder ein Anhydrid bedeutet und $R^3$ die oben zu Formel I angegebenen Bedeutungen hat, oder daß man gegebenfalls

c) das Nigericinderivat der Formel II oxidiert zu der Verbindung der Formel V

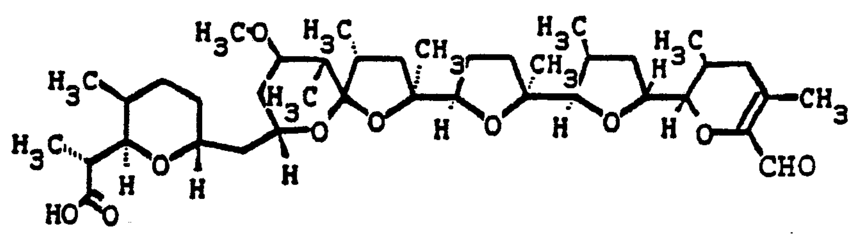

(V)

und daß man gegebenenfalls anschließend die Verbindungen der Formel I in ihre physiologisch verträglichen Salze überführt.

8. Arzneimittel, enthaltend eine Verbindung der Formel I gemäß Anspruch 1, 2 oder 3.

9. Verfahren zur Herstellung von Arzneimitteln, welche antiviral wirken, dadurch gekennzeichnet, daß man in das Arzneimittel eine Verbindung der Formel I gemäß Anspruch 1, 2 oder 3 einverleibt.

10. Verfahren zur Herstellung von Arzneimitteln, welche antibakteriell wirken, dadurch gekennzeichnet, daß man in das Arzneimittel eine Verbindung der Formel I gemäß Anspruch 1, 2 oder 3 einverleibt.

Europäisches Patentamt

# EUROPÄISCHER RECHERCHENBERICHT

Nummer der Anmeldung

EP 89115760.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | CHEMICAL ABSTRACTS, Band 83, Nr. 11, 15. September 1975, Columbus, Ohio, USA GACHON,P.; KERGOMARD,A. "Grisorixin, an ionophorous antibiotic of the nigericin group. II. Chemical and structural study of grisorixin and some derivatives" Seite 556, Spalte 2, Zusammenfassung-Nr. 96 987q & J. Antibiot. 1975,28(5), 351-7 ---- | 1,4-10 | C 07 D 493/10<br>A 61 K 31/35 |
| | | | RECHERCHIERTE SACHGEBIETE (Int. Cl.5) |
| | | | C 07 D 493/00 |

Der vorliegende Recherchenbericht wurde für alle Patentansprüche erstellt.

| Recherchenort | Abschlußdatum der Recherche | Prufer |
|---|---|---|
| WIEN | 22-11-1989 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument